(19) Europäisches Patentamt / European Patent Office / Office européen des brevets

(11) **EP 1 633 441 B1**

(12) **EUROPÄISCHE PATENTSCHRIFT**

(45) Veröffentlichungstag und Bekanntmachung des Hinweises auf die Patenterteilung:
**13.02.2008   Patentblatt 2008/07**

(21) Anmeldenummer: **04739309.5**

(22) Anmeldetag: **21.05.2004**

(51) Int Cl.:
*A61Q 19/00* (2006.01)    *A61Q 19/08* (2006.01)
*A61K 8/96* (2006.01)    *A61K 8/97* (2006.01)
*A61K 8/64* (2006.01)

(86) Internationale Anmeldenummer:
**PCT/EP2004/005543**

(87) Internationale Veröffentlichungsnummer:
**WO 2004/105717 (09.12.2004 Gazette 2004/50)**

(54) **KOSMETIKUM MIT MINERALWASSER FÜR DIE REMINERALISIERUNGS- UND ANTI-ALTERUNGSBEHANDLUNG DER HAUT**

COSMETIC PRODUCT CONTAINING MINERAL WATER FOR REMINERALISING AND REJUVENATING THE SKIN

PRODUIT COSMETIQUE CONTENANT DE L'EAU MINERALE POUR LA REMIN ERALISATION ET UN TRAITEMENT ANTI-VIEILLISSEMENT DE LA PEAU

(84) Benannte Vertragsstaaten:
**AT BE BG CH CY CZ DE DK EE ES FI FR GB GR HU IE IT LI LU MC NL PL PT RO SE SI SK TR**

(30) Priorität: **28.05.2003   DE 10325158**

(43) Veröffentlichungstag der Anmeldung:
**15.03.2006   Patentblatt 2006/11**

(73) Patentinhaber: **Coty B.V.**
**2031 CC  Haarlem (NL)**

(72) Erfinder:
• **GOLZ-BERNER, Karin**
**98000 Monaco (MC)**
• **ZASTROW, Leonhard**
**98000 Monaco (MC)**
• **BERNINI, Dorothée**
**98000 Monaco (MC)**

(74) Vertreter: **Walter, Wolf-Jürgen**
**Anwaltskanzlei**
**Gulde Hengelhaupt Ziebig & Schneider**
**Wallstrasse 58-59**
**10179 Berlin (DE)**

(56) Entgegenhaltungen:
EP-A- 1 170 002          DE-A- 10 140 538
FR-A- 2 802 413          FR-A- 2 819 718
US-A- 5 614 215          US-A- 5 690 946
US-B1- 6 426 080

• NN: "Blue Lagoon Iceland"[Online] XP002298084 Gefunden im Internet: URL:http://www.bluelagoon.com/top_en/About _Blue_ Lagoon/>
• JEAN-MICHEL KORNPROBST: "Base de données Eaux Minérales Naturelles et Eaux de Source d'origine française"[Online] XP002298085 UNIVERSITE DE NANTES Gefunden im Internet: URL:www.univ-nantes.fr/>
• LINTNER K ET AL: "BIOLOGICALLY ACTIVE PEPTIDES: FROM A LABORATORY BENCH CURIOSITY TO A FUNCTIONAL SKIN CARE PRODUCT" INTERNATIONAL JOURNAL OF COSMETIC SCIENCE, KLUWER ACADEMIC PUBLISHERS, DORDRECHT, NL, Bd. 22, 2000, Seiten 207-218, XP001183031 ISSN: 0142-5463
• NN: "Adidas Daily Energizing Face Lotion for Men" EWG REPORT SKIN DEEP, [Online] XP002298086 Gefunden im Internet: URL:http://www.ewg.org/reports/skindeep/pr oductinfo.php?prod_id=912992>

Bemerkungen:
Die Akte enthält technische Angaben, die nach dem Eingang der Anmeldung eingereicht wurden und die nicht in dieser Patentschrift enthalten sind.

EP 1 633 441 B1

**Beschreibung**

Die Erfindung betrifft eine kosmetische Zusammensetzung, die zur Remineralisierung und gegen die Alterung menschlicher Haut eingesetzt werden kann.

Die kosmetische Anwendung von Mineralwässern ist bekannt. In der EP 699432 B1 wird eine kosmetische Zusammensetzung beansprucht, die wenigstens einen Wirkstoff mit einer Reizwirkung enthält, wie Salicylsäure, $\alpha$-Hydroxysäuren, Retinoide oder Benzoylperoxid, sowie wenigstens ein linderndes Mittel, das unter Thermalwässern oder Mineralwässern mit einem Mineralstoffgehalt von wenigstens 700 mg/l und einer Gesamtkonzentration von Carbonat und Hydrogencarbonat von wenigstens 360 mg/l ausgewählt ist.

Weiterhin ist in der EP 1170002 ein Verfahren zur Verringerung des Verlustes der Hautelastizität beschrieben, das die Verwendung von Mineralwasser mit wenigstens 200 mg/l Mineralien vorsieht, davon 30-150 mg/l Ca und 10-50 mg/l Mg.

Ein Wasser aus "Blue Lagoon Iceland" (URL:http://www.bluelagoon.com/top_en/About Blue Lagoon) zeigt zwar remineralisierende Eigenschaften, enthält jedoch nur 0,6 mg/kg Mg und 11,4 4 mg $CO_2$, wobei P, Se und Zn offenbar auch in geringen Mengen nicht vorhanden sind.

Die US 5690946 beschreibt Wässer ohne Angabe von Na, K, Mg und Ca und mit einem $CO_3^{-2}$/$HCO_3^-$-Gehalt von >2 mg/l, vorzugsweise >150 mg/l, wobei P, Se und Zn ebenfalls nicht vorhanden sind.

Die EP 1170002 nennt Wässer mit Mineralisierungswerten von 400-1000 mg und sehr geringen Alkaligehalten von <10 mg/l und höheren $CO_3^{-2}$/$HCO_3^-$-Gehalten von 150-700 mg/l.

In Kornprobst,J-M., Base de données Eaux Minerales Naturelles et Eaux de Source d'origine francaise" werden eine Vielzahl französischer Mineralwässer mit Gehalten an unterschiedlichen Mineralien aufgeführt, die sich jedoch alle von denen der Erfindung unterscheiden, insbesondere weist keines dieser Wässer niedrige $CO_3^{-2}$/$HCO_3^-$-Gehalte und hohe Alkali-/Erdalkaligehalte auf.

Der Erfindung liegt die Aufgabe zugrunde, ein Kosmetikum bereitzustellen, das langanhaltende remineralisierende Eigenschaften hat. Ein weitere Aufgabe besteht darin, die remineralisierende Wirkung durch eine auffrischende und Anti-Alterungswirkung zu ergänzen.

Erfindungsgemäß ist das Kosmetikum für die Remineralisierungs- und Anti-Alterungsbehandlung der Haut, dadurch gekennzeichnet, daß es neben kosmetischen Hilfs- oder Trägerstoffen und weiteren Wirkstoffen

0,5 bis 3 Gew-% eines Wassers vulkanischen Ursprungs enthält, enthaltend.

0,01 - 0,05 mg/l Eisen

100 - 300 mg/l Kalium

1000-2000 mg/l Natrium

80 - 200 mg/l Magnesium

50 - 150 mg/l Calcium

50 bis 150 mg/l Silicium (als $SiO_2$)

0,01 bis 0,1 mg/l Phosphor

0,001 - 0,005 mg/l Selen

0,01 - 0,03 mg/l Zink.

Der Einsatz eines vulkanischen Wassers aus überirdischen vulkanischen Seen zeigt eine deutliche Verbesserung der Re-Sensibilisierung der Haut, die im Verläufe des Alterungsprozesses bei 50- bis 65-jährigen bis zu 50 % ihrer Sensibilität verliert. Dadurch wird die Barrierefunktion der Haut in der Epidermis, insbesondere im Stratum corneum verbessert. Die Feuchthaltung des Stratum corneum ist besonders wichtig, da dies die mechanischen Eigenschaften Elastizität und Plastizität der Haut bestimmt. Im Stratum corneum liegt das Wasser unter zwei thermodynamischen Formen vor: als freies Wasser, in dem verschiedene Ionen oder Moleküle gelöst sind, und als gebundenes Wasser, das mit den Lipiden und Proteinen des Stratum corneum Wechselwirkungen eingeht. Das gebundene Wasser nimmt 20-30 % des Gesamtvolumens ein. Bei trockener Haut wird neben Verlust von freiem.Wasser, das aus der Dermis über die Basalschicht an die Hautoberfläche gelangt, auch das Wasser verringert, das an das Enzym gebunden ist, das für den Corneodesmosomen-Abbau verantwortlich ist. Die Haut verliert dadurch an Plastizität und Elastizität.

Auch das empfindliche Gleichgewicht der hygroskopischen Substanzen im Stratum corneum wird gestört, die als NMF (Natural Moisturing Factor) bekannt sind und wozu eine Reihe von Ionen wie Na, K, Ca, Cl, Phosphor sowie bestimmte organische Säuren, Harnstoff und Aminosäuren gehören.

Es wurde gefunden, daß Vulkanwasser eine Anreicherung von verschiedenen Mineralstoffen in einer für die Hautsuszeptibilität annehmbaren Form und Zusammensetzung enthält und dadurch besonders geeignet ist, zur langanhaltenden Remineralisierung der Haut beizutragen. Die aus Vulkangestein über lange Zeiträume herausgelösten mineralischen Ionen wie Eisen, Selen, Zink, Calcium, Magnesium, Natrium, Kalium und Phosphor tragen als Cofaktor bei den enzymatischen biochemischen Reaktionen dazu bei, sich der natürlichen Hautstruktur in dieser Zusammensetzung auch bei älterer Haut anzunähern. Das betrifft sowohl die Na- und K-Gehalte, die für die Steuerung des Wassergehaltes bedeutsam sind, als auch Ca-, Mg- und P-Gehalte, die in Alterungs- und Wachstumsprozessen der Zellen entscheidende Funktionen haben.

Die erfindungsgemäße Zusammensetzung mit einem Wasser, das im Unterschied zu bekannten Thermal- und Mineralwassern nur sehr geringe Carbonat- und Hydrogencarbonatgehalte hat d.h. insgesamt unter 1-2 mg/l, und das sehr höhe Gehalte an Na, Mg und Si aufweist und zugleich P, Se und Zn in hautadäquaten Konzentrationen enthält, zeigt bei Elastizätsmessungen mit bekannten Verfahren (Cutometer) unerwartet hohe Werte auch nach längeren Zeiträumen.

Vorzugsweise wird ein Wasser aus einem Vulkansee im Gebiet Clermont-Ferrand (Frankreich) eingesetzt.

In einer zweiten Ausführungsform der Erfindung enthält das Kosmetikum zusätzlich als weiteren Wirkstoff 0,01 bis 2 Gew-% eines Extraktes von Meerfenchel Crithmum maritinum. Es wurde gefunden, daß Meerfenchelextrakt einen signifikante Wirkung auf die Synthese von Ceramiden hat und daß z.B. bei einer Konzentration von 1 % die Ceramidsynthese um etwa 70 % verbessert wird.

In einer dritten Ausführungsform der Erfindung enthält das Kosmetikum zusätzlich als weiteren Wirkstoff 0,01 bis 2 Gew-% einer Lösung des Peptids Palmitoyl-Gly-His-Lys in Propylenglycol, vorzugsweise in einer Kombination mit 0,01 bis 2 Gew-% eines Extraktes von Meerfenchel Crithmum maritinum. Damit wird die Synthese von Kollagen über die Fibroblasten stimuliert und ebenso die Synthese von Hyaluronsäure. Das Peptid liegt in der Lösung mit Propylenglycol in einer Konzentration von etwa 100 mg/l vor (oder 0,001 bis 0,2 % des Peptids).

In einer vierten Ausführungsform der Erfindung enthält das Kosmetikum zusätzlich als weiteren Wirkstoff 0,01 bis 5 Gew-% an hydrolysiertem Sojaprotein, vorzugsweise in einer Kombination mit 0,01 bis 2 Gew-% eines Extraktes von Meerfenchel Crithmum maritinum und 0,01 bis 2 Gew-% des Peptids Palmitoyl-Gly-His-Lys. In dieser Kombination zeigt das Kosmetikum eine über mehrere Stunden anhaltende Feuchthaltewirkung ohne Zusatz üblicher Feuchthaltemittel und zugleich eine Verringerung der kleinen Hautfältchen um etwa 35 % auch noch nach 8 Stunden.

In einer fünften Ausführungsform der Erfindung enthält das Kosmetikum zusätzlich als weiteren Wirkstoff ein Gemisch von Pflanzenextrakten auf alkoholischer Basis, bestehend aus 0,2 Gew-% Extrakt grüner Kaffeebohnen, 0,2 Gew-% Extrakt von Blättern von Camellia sinensis, 0,2 Gew-% Extrakt von Pongamia pinnata und 0,2 Gew-% Extrakt der Wurzeln von Angelica archangelica und 99,2 Gew-% Ethanol. Dieses Gemisch, dessen Wirkstoffe nicht verkapselt in Liposmen sind, kann in einem Anteil von 0,1 bis 2 Gew-% in dem Kosmetikum enthalten sein, bezogen auf das Gesamtgewicht des Kosmetikums. Es wurde gefunden, daß ein solches Wirkstoffgemisch einen unerwartet hohen Radikalschutzfaktor (RPF) von etwa 1800 Radikale pro mg zeigt, gemessen durch Bestimmung der Anzahl freier Radikale einer Lösung einer Testsubstanz ($S_1$) mittels Elektronenspinresonanz (ESR) im Vergleich mit dem ESR-Meßergebnis der kosmetischen Wirkstoffzubereitung nach der Beziehung

$$RPF = (RC \times RF) / PI$$

worin $RF = (S_1 - S_2) / S_1$ ; RC = Konzentration der Testsubstanz (Radikale/ml); PI = Konzentration der Wirkstoffzubereitung (mg/ml) (Messung gemäß WO 99/66881).

Das erfindungsgemäße Kosmetikum enthält weiterhin bestimmte kosmetische Hilfs- und Trägerstoffe, wie sie üblicherweise in solchen Zubereitungen verwendet werden, z.B. Wasser, Konservierungsmittel, Farbstoffe, Pigmente mit färbender Wirkung, Verdickungsmittel, Duftstoffe" Alkohole, Polyole, Ester, Elektrolyte, Gelbildner, Siliconöle, Polymere, Copolymere, Emulgatoren, Stabilisatoren.

Zu den kosmetischen Wirkstoffen, die ebenfalls enthalten sein können, gehören z. B. anorganische und organische Lichtschutzmittel, Feuchthaltemittel, Enzyme und weitere pflanzliche Wirkstoffe.

Besonders bevorzugte kosmetische Zusammensetzungen sind solche, die in Form eines Feuchtigkeits-Hautbalsams vorliegen, der 0,08 bis 1,4 Gew-% Wasser vulkanischen Ursprungs, 0,2 bis 0,8 Gew-% eines Extraktes von Meerfenchel Crithmum maritinum, 0,3 bis 0,9 Gew-% des Peptids Palmitoyl-Gly-His-Lys und 0,8 bis 1,25 Gew-% an hydrolysiertem Sojaprotein enthält und weiterhiri enthält 1-4 Gew-% Glycerin, 1-5 Gew-% Butylenglycol oder Propylenglycol, 2-4 Gew-% Cetearylalkohol, 6-9 Gew-% Dicaprylcarbonat, 0,5-1,3 Gew-% Phenoxyethanol, 0,1-0,5 Gew-% Chlorphenesin, 2,5-4 Gew-% Beheneth-25, 6-9 Gew-% Shea Butter, 2-4 Gew-% modifizierten Maisstärkepuder, 2,5-4 Gew-% Simulgel NS, 1-3,5 Gew-% Dimethicone, 0,05-0,5 Gew-% Farbstoffe, 0,05-0,2 Gew-% organische Sonnenschutzfilter für die Farbstoffe, 0,1-0,5 Gew-% Konservierungsmittel, 0,05-0,2 Gew-% Tetranatrium-ethylendiaminessigsäure sowie 0,1-0,5 Gew-% eines alkoholischen Pflanzenextraktgemisches aus 0,2 Gew-% Samen von grünen Kaffeebohnen, 0,2 Gew-% Camellia sinensis-Blättern, 0,2 Gew-% Ponagamia pinnata, 0,2 Gew-% Angelikawurzel und 99,8 Gew-% Ethanol. Der Rest zu 100 Gew-% ist destilliertes Wasser.

Weitere besonders bevorzugte kosmetische Zusammensetzungen sind solche, die in Form eines hochfeuchten Gels für normale und ölige Haut vorliegt, das 0,08 bis 1,4 Gew-% Wasser vulkanischen Ursprungs, 0,2 bis 0,8 Gew-% eines Extraktes von Meerfenchel Crithmum maritinum, 0,3 bis 0,9 Gew-% des Peptids Palmitoyl-Gly-His-Lys und 0,8 bis 1,25 Gew-% an hydrolysiertem Sojaprotein enthält, und das weiterhin enthält 10-14 Gew-% Cyclomethicone, 0,5-2 Gew-% Phenyl Trimethicone, 1-5 Gew-% Butylenglycol oder Propylenglycol, 3-5 Gew-% Ethanol, 0,1-0,5 Gew-% Chlorphenesin, 2-4 Gew-% Puder aus Methyl Methacrylate Crosspolymer, 2,5-4 Gew-% Simulgel NS, 1-3,5 Gew-% Dimethicone, 0,05-0,5 Gew-% Farbstoffe, 0,1-0,5 Gew-% Konservierungsmittel, 0,1-0,5 Gew-% des zuvor genannten alkoholischen Pflanzenextraktgemisches, 0,1-0,5 Gew-% Parfüm und den Rest zu 100 Gew-% dest. Wasser.

Die Erfindung soll nachstehend durch Beispiele näher erläutert werden. Alle Angaben erfolgen in Gewichtsprozent, sofern nichts anderes angegeben ist.

Beispiel 1 **Feuchtigkeits-Hautbalsam**

| Phase A | |
|---|---|
| Wasser | q.s. ad 100 |
| Glycerin | 2,0 |
| Butylenglycol | 2,0 |
| Tetranatrium-ethylendiaminessigsäure | 0,1 |
| Konservierungsmittel | 0,4 |
| pH-Regulator | 0,3 |
| **Phase B** | |
| Beheneth-25 | 3,3 |
| Cetearylalkohol | 2,7 |
| Dicaprylcarbonat | 8,5 |
| Shea Butter | 7,2 |
| Phenoxyethanol | 0,9 |
| modifizierter Maisstärkepuder | 3,0 |
| Dimethicone | 1,4 |
| Simulgel® NS | 3,5 |
| **Phase C** | |
| Farben | 0,1 |
| Wasser vulkanischen Ursprungs | 1,0 |
| Peptid Palmitoyl-Gly-His-Lys | 0,5 |
| alkoholisches Pflanzenextraktgemisch* | 0,2 |
| Crithmum maritinum-Extrakt | 0,5 |
| hydrolysiertes Sojaprotein | 1,0 |
| Benzophenone-4 (für Farben) | 0,4 |
| * aus 0,2 Gew-% Kaffeebohnensamen, 0,2 Gew-% Camellia sinensis Blättern, 0,2 Gew-% Ponagamia pinnata, 0,2 Gew-% Angelikawurzel und 99,8 Gew-% Ethanol. | |

Die Phasen A und B werden separat bei etwa 60°C gemischt und mit der bei ca. 35°C gemischten Phase C unter Rühren bei etwa 35°C zusammengeführt.

Beispiel 2 **Feuchtigkeits-Gel**

| Phase A | |
|---|---|
| Wasser | q.s. ad 100 |
| Simulgel® NS | 3,2 |
| Propylenglycol | 4,0 |
| pH-Regulator | 0,3 |
| Methyl Methacrylate Crosspolymer | 1,2 |

(fortgesetzt)

| | |
|---|---|
| **Phase B** | |
| Cyclomethicone | 11,5 |
| Dimethicone | 3,0 |
| Phenyl Trimethicone | 1,0 |
| Peptid Palmitoyl-Gly-His-Lys | 0,5 |
| **Phase C** | |
| Ethanol | 4,0 |
| **Phase D** | |
| Farben | 0, 3 |
| Parfüm | 0,2 |
| Konservierungsmittel. | 0,3. |
| Wasser vulkanischen Ursprungs | 1,0 |
| alkoholisches Pflanzenextraktgemisch* | 0,2 |
| Crithmum maritinum-Extrakt | 0,5 |
| hydrolysiertes Sojaprotein | 1,0 |
| * aus 0,2 Gew-% Kaffeebohnensamen, 0,2 Gew-% Camellia sinensis Blättern, 0,2 Gew-% Ponagamia pinnata, 0,2 Gew-% Angelikawurzel und 99,8 Gew-% Ethanol. | |

Die Phasen A und B werden separat bei etwa 60°C gemischt und mit den bei ca. 35°C gemischten Phasen C und D unter Rühren bei etwa 35°C zusammengeführt.

Beispiel 3 **Elastizitätsmessungen**

Es wurde die Viskoelastizität der Haut mit einem Cutometer SEM 575® (Courage+Khazaka, Köln, Deutschland) gemessen mit einem Saugdruck von 500 mbar. Eine Hautfläche von 2 mm Durchmesser wurde für 10 Sek. mit konstantem Saugdruck angesaugt und wieder drucklos gemacht und dabei die Penetrationstiefe der Haut gemessen. Das Verhältnis $U_v/U_e$ (viskolelastische Rückkehr nach elastischer Deformation/elastische Deformation der Haut bei Anlegen des Vakuums) wurde bestimmt.

Bei einer Gruppe von 18 Probanden wurde im Zeitraum von 3 Wochen im Wadenbereich einmal täglich eine Messung durchgeführt, nachdem die Probanden zuvor 20 Min. bei 22°C $\pm$ 2,5°C und 30 % relativer Luftfeuchtigkeit akklimatisiert wurden.

Der auf einer Wade zweimal täglich aufgestrichene Balsam gemäß Beispiel 1 (Probe A) zeigte gegenüber einem ebenfalls zweimal täglich aufgestrichenen Balsam ohne die Phase C gemäß Beispiel 1 (Probe B) bereits nach 3 Tagen eine Erhöhung des Verhältnisses $U_v/U_e$ von 10 %, nach 6 Tagen von 18 % und nach 14 Tagen von 22 %. Nach 3 Wochen zeigte die Kurve von Probe A eine Annäherung an eine Asymptote, während die Kurve von Probe abfiel. Damit lagen die Werte nach 3 Wochen von Probe B um 25 % über Probe B, woraus die langandauernde Wirkung der erfindungsgemäßen Zusätze deutlich hervorgeht.

**Patentansprüche**

1. Kosmetikum für die Remineralisierungs- und Anti-Alterungsbehandlung der Haut, **dadurch gekennzeichnet, daß** es neben kosmetischen Hilfs- oder Trägerstoffen und weiteren Wirkstoffen 0,5 bis 3 Gew-% eines Wassers vulkanischen Ursprungs enthält, enthaltend

   0,01 - 0,05 mg/l Eisen
   100 - 300 mg/l Kalium
   1000-2000 mg/l Natrium
   80 - 200 mg/l Magnesium
   50 - 150 mg/l Calcium
   50 bis 150 mg/l Silicium (als $SiO_2$)
   0,01 bis 0,1 mg/l Phosphor
   0,001 - 0,005 mg/l Selen

0,01 - 0,03 mg/l Zink
unter 1-2 mg/l Carbonat und Hydrogencarbonat.

**2.** Kosmetikum nach Anspruch 1, **dadurch gekennzeichnet, daß** es als weiteren Wirkstoff 0,01 bis 2 Gew-% eines Extraktes von Meerfenchel <u>Crithmum maritinum</u> enthält.

**3.** Kosmetikum nach Anspruch 1, **dadurch gekennzeichnet, daß** es als weiteren Wirkstoff 0,01 bis 2 Gew-% eines Extraktes von Meerfenchel <u>Crithmum maritinum</u> in Kombination mit 0,01 bis 2 Gew-% einer Lösung des Peptids Palmitoyl-Gly-His-Lys in Propylenglycol enthält.

**4.** Kosmetikum nach Anspruch 1, **dadurch gekennzeichnet, daß** es als weiteren Wirkstoff 0,01 bis 2 Gew-% eines Extraktes von Meerfenchel Crithmum maritinum in Kombination mit 0,01 bis 2 Gew-% des Peptids Palmitoyl-Gly-His-Lys und 0,01 bis 5 Gew-% hydrolysiertem Sojaprotein enthält.

**5.** Kosmetikum nach Anspruch 1, **dadurch gekennzeichnet, daß** das Wasser vulkanischen Ursprungs ein Oberflächenwasser aus einem Vulkansee ist.

**6.** Kosmetikum nach Anspruch 1, **dadurch gekennzeichnet, daß** das Wasser vulkanischen Ursprungs ein unterirdisches Wasser aus der unmittelbaren Nähe eines Vulkans ist.

**7.** Kosmetikum nach Anspruch 1, **dadurch gekennzeichnet, daß** es ein Wasser vulkanischen Ursprungs enthält, enthaltend
0,025 - 0,05 mg/l Eisen
170 - 300 mg/l Kalium
1600 - 2000 mg/l Natrium
130 - 200 mg/l Magnesium
90 - 150 mg/l Calcium
90 bis 150 mg/l Silicium (als $SiO_2$)
0,06 bis 0,1 mg/l Phosphor
0,003 - 0,005 mg/l Selen
0,02 - 0,03 mg/l Zink.

**8.** Kosmetikum nach Anspruch 4, **dadurch gekennzeichnet, daß** es ein Feuchtigkeits-Hautbalsam ist, der 0,08 bis 1,4 Gew-% Wasser vulkanischen Ursprungs, 0,2 bis 0,8 Gew-% eines Extraktes von Meerfenchel <u>Crithmum maritinum,</u> 0,3 bis 0,9 Gew-% des Peptids Palmitoyl-Gly-His-Lys und 0,8 bis 1,25 Gew-% an hydrolysiertem Sojaprotein enthält und weiterhin enthält
1-4 Gew-% Glycerin
1-5 Gew-% Butylenglycol oder Propylenglycol
2-4 Gew-% Cetearylalkohol
6-9 Gew-% Dicaprylcarbonat
0,5-1,3 Gew-% Phenoxyethanol
0,1-0,5 Gew-% Chlorphenesin
2,5-4 Gew-% Beheneth-25
6-9 Gew-% Shea Butter
2-4 Gew-% modifizierten Maisstärkepuder
2,5-4 Gew-% Simulgel NS
1-3,5 Gew-% Dimethicone
0,05-0,5 Gew-% Farbstoffe
0,05-0,2 Gew-% organische Sonnenschutzfilter für die Farbstoffe
0,1-0,5 Gew-% Konservierungsmittel
0,1-0,5 Gew-% eines alkoholischen Pflanzenextraktgemisches aus
0,2 Gew-% Kaffeesamen,
0,2 Gew-% Camellia sinensis-Blättern,
0,2 Gew-% Ponagamia pinnata,
0,2 Gew-% Angelikawurzel und
99,8 Gew-% Ethanol
0,05-0,2 Gew-% Tetranatrium-ethylendiaminessigsäure
Rest zu 100 Gew-% dest. Wasser.

**9.** Kosmetikum nach Anspruch 4, **dadurch gekennzeichnet, daß** es ein Gel ist, das 0,08 bis 1,4 Gew-% Wasser vulkanischen Ursprungs, 0,2 bis 0,8 Gew-% eines Extraktes von Meerfenchel <u>Crithmum maritinum,</u> 0,3 bis 0,9 Gew-% des Peptids Palmitoyl-Gly-His-Lys und 0,8 bis 1,25 Gew-% an hydrolysiertem Sojaprotein enthält, und das weiterhin enthält

10-14 Gew-% Cyclomethicone
0,5-2 Gew-% Phenyl Trimethicone
1-5 Gew-% Butylenglycol oder Propylenglycol
3-5 Gew-% Ethanol
0,1-0,5 Gew-% Chlorphenesin
2-4 Gew-% Puder aus Methyl Methacrylate Crosspolymer
2,5-4,Gew-% Simulgel NS
1-3,5 Gew-% Dimethicone
0,05-0,5 Gew-% Farbstoffe
0,1-0,5 Gew-% Konservierungsmittel
0,1-0,5 Gew-% eines alkoholischen Pflarizenextraktgemisches aus
0,2 Gew-% Kaffeesamen,
0,2 Gew-% Camellia sinensis-Blättern,
0,2 Gew-% Ponagamia pinnata,
0,2 Gew-% Angelikawurzel und 99,8 Gew-% Ethanol
0,1-0,5 Gew-% Parfüm
Rest zu 100 Gew-% dest. Wasser.

**Claims**

**1.** A cosmetic for remineralising and rejuvenating the skin which, in addition to cosmetic auxiliaries or carriers and further active agents, comprises 0.5 to 3% by weight water of volcanic origin containing
0.01-0.05 mg/l iron
100-300 mg/l potassium
1,000-2,000 mg/l sodium
80-200 mg/l magnesium
50-150 mg/l calcium
50-150 mg/l silicon (as $SiO_2$)
0.01-0.1 mg/l phosphorus
0.001-0.005 mg/l selenium
0.01-0.03 mg/l zinc
below 1-2 mg/l carbonate and hydrogen carbonate.

**2.** A cosmetic according to Claim 1, wherein said cosmetic contains 0.01 to 2% by weight of an extract from samphire Crithmum maritimum as an additional active agent.

**3.** A cosmetic according to Claim 1, wherein said cosmetic contains 0.01 to 2% by weight of an extract from samphire Crithmum maritimum as an additional active agent, in combination with 0.01 to 2% by weight of a solution of the peptide palmitoyl-gly-his-lys in propylene glycol.

**4.** A cosmetic according to Claim 1, wherein said cosmetic contains 0.01 to 2% by weight of an extract from samphire Crithmum maritimum as an additional active agent, in combination with 0.01 to 2% by weight of the peptide palmitoyl-gly-his-lys and 0.01 to 5% by weight hydrolysed soy protein.

**5.** A cosmetic according to Claim 1, wherein said water of volcanic origin is surface water from a volcanic lake.

**6.** A cosmetic according to Claim 1, wherein said water of volcanic origin is underground water from the immediate vicinity of a volcano.

**7.** A cosmetic according to Claim 1, wherein said cosmetic contains water of volcanic origin containing
0.025-0.05 mg/l iron
170-300 mg/l potassium
1,600-2,000 mg/l sodium

130-200 mg/l magnesium
90-150 mg/l calcium
90-150 mg/l silicon (as $SiO_2$)
0.06-0.1 mg/l phosphorus
0.003-0.005 mg/l selenium
0.02-0.03 mg/l zinc.

8. A cosmetic according to Claim 4, wherein said cosmetic is a moisturising skin balm containing 0.08 to 1.4% by weight water of volcanic origin, 0.2 to 0.8% by weight of an extract from samphire Crithmum maritimum, 0.3 to 0.9% by weight of the peptide palmitoyl-gly-his-lys and 0.8 to 1.25% by weight hydrolysed soy protein, and which further contains
1-4% by weight glycerine
1-5% by weight butylene glycol or propylene glycol
2-4% by weight cetearyl alcohol
6-9% by weight dicapryl carbonate
0.5-1.3% by weight phenoxyethanol
0.1-0.5% by weight chlorphenesin
2.5-4% by weight Beheneth-25
6-9% by weight shea butter
2-4% by weight modified maize starch powder
2.5-4% by weight Simulgel NS
1-3.5% by weight dimethicone
0.05-0.5% by weight colourants
0.05-0.2% by weight organic sunscreens for the colourants
0.1-0.5% by weight preservative
0.1-0.5% by weight of a mixture of alcoholic extracts from plants consisting of
0.2% by weight coffee seeds,
0.2% by weight Camellia sinensis leaves,
0.2% by weight Ponagamia pinnata,
0.2% by weight angelica root, and
99.2% by weight ethanol
0.05-0.2% by weight tetrasodium ethylenediamine tetraacetic acid
remainder up to 100% by weight distilled water.

9. A cosmetic according to Claim 4, wherein said cosmetic is a gel containing 0.08 to 1.4% by weight water of volcanic origin, 0.2 to 0.8% by weight of an extract from samphire Crithmum maritimum, 0.3 to 0.9% by weight of the peptide palmitoyl-gly-his-lys and 0.8 to 1.25% by weight hydrolysed soy protein, and which further contains
10-14% by weight cyclomethicone
0.5-2% by weight phenyl trimethicone
1-5% by weight butylene glycol or propylene glycol
3-5% by weight ethanol
0.1-0.5% by weight chlorphenesin
2-4% by weight powdered methyl methacrylate crosspolymer
2.5-4% by weight Simulgel NS
1-3.5% by weight dimethicone
0.05-0.5% by weight colourants
0.1-0.5% by weight preservative
0.1-0.5% by weight of a mixture of alcoholic extracts from plants consisting of
0.2% by weight coffee seeds,
0.2% by weight Camellia sinensis leaves,
0.2% by weight Ponagamia pinnata,
0.2% by weight angelica root and 99.2% by weight ethanol
0.1-0.5% by weight perfume
remainder up to 100% by weight distilled water.

**Revendications**

1. Produit cosmétique pour le traitement par reminéralisation et pour le traitement anti-vieillissement de la peau, **caractérisé en ce qu'**il contient, outre des adjuvants et des excipients cosmétiques et autres principes actifs 0,5 à 3 % en poids d'une eau d'origine volcanique, contenant

   0,01 - 0,05 mg/l de fer
   100 - 300 mg/l de potassium
   1000 - 2000 mg/l de sodium
   80 - 200 mg/l de magnésium
   50 - 150 mg/l de calcium
   50 à 150 mg/l de silicium (comme $SiO_2$)
   0,01 à 0,1 mg/l de phosphore
   0,001 - 0,005 mg/l de sélénium
   0,01 - 0,03 mg/l de zinc
   moins de 1 - 2 mg/l de carbonate et d'hydrocarbonate.

2. Produit cosmétique selon la revendication 1, **caractérisé en ce qu'**il contient comme autre principe actif 0,01 à 2 % en poids d'un extrait de criste marine *(crithmum maritimum).*

3. Produit cosmétique selon la revendication 1, **caractérisé en ce qu'**il contient comme autre principe actif 0,01 à 2 % en poids d'un extrait de criste marine (*crithmum maritimum*) combiné à 0,01 à 2 % en poids du peptide palmitoyl-glys-his-lys en dissolution dans du glycol propylique.

4. Produit cosmétique selon la revendication 1, **caractérisé en ce qu'**il contient comme autre principe actif 0,01 à 2 % en poids d'un extrait de criste marine (*crithmum maritimum*) combiné à 0,01 à 2 % en poids du peptide palmitoyl-glys-his-lys et à 0,01 à 5 % en poids d'une protéine de soja hydrolysée.

5. Produit cosmétique selon la revendication 1, **caractérisé en ce que** l'eau d'origine volcanique est une eau de surface d'un lac volcanique.

6. Produit cosmétique selon la revendication 1, **caractérisé en ce que** l'eau d'origine volcanique est une eau souterraine se trouvant à proximité immédiate d'un volcan.

7. Produit cosmétique selon la revendication 1, **caractérisé en ce qu'**il contient une eau d'origine volcanique, contenant

   0,025 - 0,05 mg/l de fer
   170 - 300 mg/l de potassium
   1600 - 2000 mg/l de sodium
   130 - 200 mg/l de magnésium
   90 - 150 mg/l de calcium
   90 à 150 mg/l de silicium (comme $SiO_2$)
   0,06 à 0,1 mg/l de phosphore
   0,003 - 0,005 mg/l de sélénium
   0,02 - 0,03 mg/l de zinc.

8. Produit cosmétique selon la revendication 4, **caractérisé en ce qu'**il constitue un baume hydratant pour la peau, lequel contient 0,08 à 1,4 % en poids d'eau d'origine volcanique, 0,2 à 0,8 % en poids d'un extrait de criste marine (*crithmum maritimum*), 0,3 à 0,9 % en poids du peptide palmitoyl-gly-his-lys et 0,8 à 1,25 % en poids d'une protéine de soja hydrolysée, et **en ce qu'**il contient en outre

   1 - 4 % en poids de glycérine
   1 - 5 % en poids de butylène glycol ou de propylène glycol
   2 - 4% en poids d'alcool de cétéaryle
   6 - 9 % en poids de dicapryl carbonate
   0,5 - 1,3 % en poids de phénoxyéthanol
   0,1 - 0,5 % en poids de chlorphénésine
   2,5 - 4% en poids de beheneth-25
   6 - 9 % en poids de beurre de carité
   2 - 4 % en poids de poudre d'amidon de maïs modifié
   2,5 - 4 % en poids de simulgel NS

1 - 3,5 % en poids de diméthicone

0,05 - 0,5 % en poids de colorants

0,05 - 0,2 % en poids de filtres de protection solaire organiques pour les colorants

0,1 - 0,5 % en poids d'agents conservateurs

0,1 - 0,5 % en poids d'un mélange alcoolique d'extraits de plantes composé de

0,2 % en poids de graines de café

0,2 % en poids de feuilles de *camellia sinensis*

0,2 % en poids de *pongamia pinnata*

0,2 % en poids de racines d'angélique et

99,8 % en poids d'éthanol

0,05 - 0, 2 % en poids d'acide acétique de tétrasodium-éthylène diamine

pour le reste jusqu'à 100 % en poids d'eau distillée.

9. Produit cosmétique selon la revendication 4, **caractérisé en ce qu'**il est un gel, qui contient 0,08 à 1,4 % en poids d'eau d'origine volcanique, 0,2 à 0,8 % en poids d'un extrait de criste marine (*crithmum maritimum*), 0,3 à 0,9 % en poids du peptide palmitoyl-gly-his-lys et 0,8 à 1,25 % en poids d'une protéine de soja hydolysée et qui contient en outre

10 - 14 % en poids de cyclométhicone

0,5 - 2 % en poids de phényl triméthicone

1 - 5 % en poids de butylène glycol ou de propylène glycol

3 - 5 % en poids d'éthanol

0,1 - 0,5 % en poids de chlorphénésine

2 - 4 % en poids de poudre composée de méthyl, de méthacrylate, de crosspolymère

2,5 - 4 % en poids de simulgel NS

1 - 3,5 % en poids de diméthicone

0,05 - 0,5 % en poids de colorants

0,1 - 0,5 % en poids d'agents conservateurs

0,1 - 0,5 % en poids d'un mélange alcoolique d'extraits de plantes composé de

0,2 % en poids de graines de café

0,2 % en poids de feuilles de *camellia sinensis*

0,2 % en poids de *pongamia pinnata,*

0,2 % en poids de racines d'angélique et 99, % en poids d'éthanol

0,1 - 0,5 % en poids de parfum

pour le reste jusqu'à 100 % en poids d'eau distillée.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 699432 B1
- EP 1170002 A
- US 5690946 A
- WO 9966881 A